# EUROPEAN PATENT SPECIFICATION

(11) **EP 3 975 946 B1**
(45) Date of publication and mention of the grant of the patent: **06.12.2023**
(21) Application number: 20730847.9
(22) Date of filing: 15.05.2020
(51) Int. Cl.: A61F 5/453, A61F 5/455

(54) **FLUID COLLECTION DEVICES, FLUID COLLECTION SYSTEMS INCLUDING THE SAME, AND METHODS OF USING THE SAME**
FLÜSSIGKEITSSAMMELVORRICHTUNGEN, FLÜSSIGKEITSAMMELSYSTEME DAMIT UND VERFAHREN ZUR VERWENDUNG DAVON
DISPOSITIFS DE COLLECTE DE LIQUIDE, SYSTÈMES DE COLLECTE DE LIQUIDE LES COMPRENANT, ET LEURS PROCÉDÉS D'UTILISATION

(30) Priority: 28.05.2019 US 201962853279 P
(43) Date of publication of application: 06.04.2022
(73) Proprietor: Purewick Corporation, El Cajon, California 92020 (US)
(72) Inventor: METZGER, Stephanie, Oyster Bay, New York 11771 (US); SCALF, Candace, Springboro, Ohio 45066 (US); OTTINGER, Caroline, North Bend, Washington 98045 (US); JOHANNES, Ashley Marie, Atlanta, Georgia 30340 (US); CHALLA, Pranav, Atlanta, Georgia 30308 (US)
(74) Representative: Hoffmann Eitle
(86) International application number: PCT/US2020/033122
(87) International publication number: WO 2020/242790

(56) References cited:
- GB-A- 1 011 517
- US-A1- 2010 234 820
- US-A1- 2019 038 451
- US-B2- 6 918 899

## Description

### BACKGROUND

An individual may have limited or impaired mobility such that typical urination processes are challenging or impossible. For example, the individual may experience or have a disability that impairs mobility. The individual may have restricted travel conditions such as those experienced by pilots, drivers, and workers in hazardous areas. Additionally, sometimes urine collection is needed for monitoring purposes or clinical testing.

Urinary catheters, such as a Foley catheter, may be used to address some of these circumstances, such as incontinence. However, urinary catheters may be uncomfortable, painful, and may lead to complications, such as infections. In another embodiment, bed pans, which are receptacles used for the toileting of bedridden patients, such as those in a health care facility, are sometimes used to address some of these circumstances. However, bed pans may be prone to discomfort, spills, and other hygiene issues.

When the individual is a male, condom-style catheters may be used to address some of the circumstances disclosed above. Condom-style catheters may include a fluid impermeable shell defining a chamber and an opening. The opening and the chamber may be configured to have the male penis inserted through the hole such that the urethral opening of the individual is disposed in the chamber.

US 2010/0234820 A1 discloses a body interface device for a urine aspiration system. A skin contact pad is adhered to the skin with a gel adhesive. A cover over the pad defines a urine-receiving chamber out of which urine is aspirated by a flexible conduit. The pad has a pear shape and the conduit is at the narrow end of the pear shape. This document shows the preamble of independent claim 1.

### SUMMARY

The invention is defined in claim 1, below. The dependent claims are directed to optional features and preferred embodiments. In an embodiment, a fluid collection device is disclosed. The fluid collection device includes a fluid impermeable barrier defining a chamber, an opening, and an outlet. The fluid collection device also includes a fluid permeable membrane extending across the opening. The fluid collection device further includes a fluid receiving portion including a portion of the fluid impermeable barrier that at least partially defines the opening and an longitudinal portion extending from the fluid receiving portion. The fluid collection device exhibits a maximum length measured from a distal tip of the longitudinal portion to an opposing portion of the fluid receiving portion. The fluid receiving portion exhibits a first maximum width measured perpendicular to the maximum length, and the longitudinal portion exhibits a second maximum width measured parallel to the first maximum width. The first maximum width is greater than the second maximum width.

In an embodiment, a fluid collection system is disclosed. The fluid collection system includes a fluid collection device. The fluid collection device includes a fluid impermeable barrier defining a chamber, an opening, and an outlet. The fluid collection device also includes a fluid permeable membrane extending across the opening. The fluid collection device further includes a fluid receiving portion including a portion of the fluid impermeable barrier that at least partially defines the opening and an longitudinal portion extending from the fluid receiving portion. The fluid collection device exhibits a maximum length measured from a distal tip of the longitudinal portion to an opposing portion of the fluid receiving portion. The fluid receiving portion exhibits a first maximum width measured perpendicular to the maximum length, and the longitudinal portion exhibits a second maximum width measured parallel to the first maximum width. The first maximum width is greater than the second maximum width. a fluid collection container in fluid communication with and positioned downstream from the fluid collection device. The fluid collection system also includes a fluid collection container in fluid communication with and positioned downstream from the fluid collection device. The fluid collection system further includes a suction source in fluid communication with the fluid collection device and the fluid collection container. The fluid collection system additionally includes at least one tube that allows the fluid collection device to be in fluid communication with the fluid collection container and the suction source.

In an embodiment, a method is disclosed. The method includes providing a fluid collection device. The fluid collection device includes a fluid impermeable barrier defining a chamber, an opening, and an outlet. The fluid collection device also includes a fluid permeable membrane extending across the opening. The fluid collection device further includes a fluid receiving portion including a portion of the fluid impermeable barrier that at least partially defines the opening and an longitudinal portion extending from the fluid receiving portion. The fluid collection device exhibits a maximum length measured from a distal tip of the longitudinal portion to an opposing portion of the fluid receiving portion. The fluid receiving portion exhibits a first maximum width measured perpendicular to the maximum length, and the longitudinal portion exhibits a second maximum width measured parallel to the first maximum width. The first maximum width is greater than the second maximum width. The method also includes positioning the opening of the fluid collection device adjacent to a urethral opening of an individual. The method further includes positioning the longitudinal portion against a perineum and/or upper thighs of the individual.

Features from any of the disclosed embodiments may be used in combination with one another, without limitation. In addition, other features and advantages of the present disclosure will become apparent to those of ordinary skill in the art through consideration of the following detailed description and the accompanying drawings.

### BRIEF DESCRIPTION OF THE DRAWINGS

The drawings illustrate several embodiments of the present disclosure, wherein identical reference numerals refer to identical or similar elements or features in different views or embodiments shown in the drawings.
**FIG. 1A** is a top plan view of a portion of a fluid collection system that includes a fluid collection device, according to an embodiment.
**FIG. 1B** is a cross-sectional view of the fluid collection system, according to an embodiment.
**FIG. 2** is a top plan view of a portion of a fluid collection system, according to an embodiment.
**FIG. 3** is excised.
**FIG. 4** is a front view of a urine collection system worn by an individual, according to an embodiment.
**FIG. 5** is a schematic illustration of a fluid collection system, according to an embodiment.
**FIG. 6** is a flow diagram of a method to use any of the fluid collection devices and/or fluid collection systems disclosed herein, according to an embodiment.

### DETAILED DESCRIPTION

Urinary collection devices, urinary collection systems including the same, and methods of using the same are disclosed herein. An example urinary collection device includes a fluid impermeable barrier defining a chamber, an opening, and an outlet. The opening and the outlet enable fluid to enter and leave the chamber, respectively. The urinary collection device also includes a fluid permeable membrane extending across the opening. The fluid permeable membrane may restrict or prevent a penis from entering the chamber. The urinary collection device includes a fluid receiving portion that includes the portions of the fluid impermeable barrier that defines the opening. The fluid receiving portion is configured to be positioned such that the opening is disposed adjacent to a urethral opening of the individual (e.g., a buried penis). The urinary collection device also includes a longitudinal portion (e.g., elongated portion) extending from the fluid receiving portion. The longitudinal portion is configured to be positioned adjacent to or near the perineum of the individual (*e*.*g*., positioned between the thighs of the individual). The urinary collection device exhibits a maximum length measured from a distal tip of the longitudinal portion to an opposing portion of the fluid receiving portion.

To facilitate their respective functions, the fluid receiving portion and the longitudinal portion exhibit different widths that are measured perpendicularly relative to the maximum length of the urinary collection device. For example, the fluid receiving portion exhibits a width that is generally greater than the width of the longitudinal portion. For instance, the fluid receiving portion exhibits a first maximum width and the longitudinal portion exhibits a second maximum width, and wherein the first maximum width is greater than the second maximum width. However, it is noted that portions of the fluid receiving portion, such as at or near a distal portion of the fluid receiving portion, may exhibit a width that is smaller than the longitudinal portion. The relatively larger width of the fluid receiving portion facilitates the opening receiving substantially all of the fluid (*e.g.,* urine, sweat, and other bodily fluids) that is discharged from or around the urethral opening of the individual. The relatively smaller width of the longitudinal portion allows the longitudinal portion to be more comfortably positioned at or near the perineum of the individual.

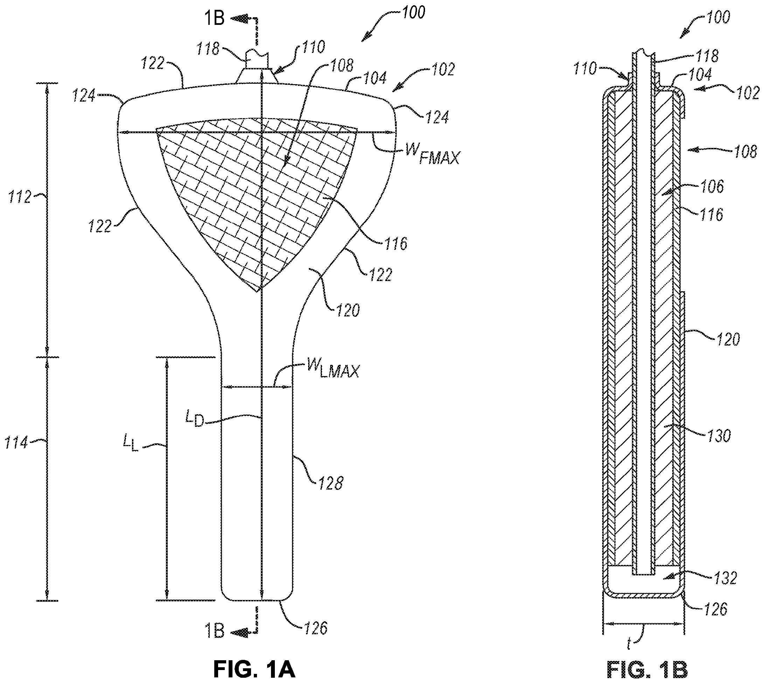

In an embodiment, the fluid receiving portion and the longitudinal portion may be distinguishable from each other by their respective widths. In an example, the width of the fluid collection device changes abruptly or substantially abruptly between the fluid receiving portion and the longitudinal portion. In an example, a rate that the widths of the fluid collection device changes between the fluid receiving portion and the longitudinal portion changes. In such an example, for instance, the width of the fluid receiving portion may be changing at or near an intersection of the fluid receiving portion (*e.g.,* decreasing with increasing proximity to the intersection) while the width of the longitudinal portion may be substantially constant at or near the intersection of the fluid receiving portion. In an embodiment, the fluid receiving portion and the longitudinal portion are distinguishable from each other because the fluid receiving portion and the longitudinal portion exhibit different shapes. The fluid receiving portion exhibits a generally triangular shape (**FIG. 1A**), while the longitudinal portion exhibits a longitudinal shape (*e.g.,* an oblong shape) that extends from the fluid receiving portion. In an embodiment, the fluid receiving portion and the longitudinal portion are distinguishable because the fluid receiving portion exhibits a size (*e.g.,* width) that is too large to be comfortably positioned at and/or near the perineum of the individual while the longitudinal portion exhibits a size that allows the longitudinal portion to be comfortably positioned at and/or near the perineum. In an embodiment, the fluid receiving portion and the longitudinal portion are distinguishable because the fluid receiving portion defines substantially all (*e.g.,* all) of the opening while the longitudinal portion does not.

In an embodiment, the fluid collection devices disclosed herein are configured to receive urine from a buried penis of a male individual. For example, as previously discussed, it may be difficult or impossible to use condom-style catheters with a buried penis. However, the fluid receiving portion of the fluid collection device may be positioned adjacent to the buried penis and the large width of the fluid receiving portion allows the opening to receive substantially all, if not all, of the bodily fluids discharged from or around the buried penis. For instance, the large width of the fluid receiving portion may allow the opening to receive urine even when the buried penis is angled relative to the fluid receiving portion and the large surface area of the opening (that is partially caused by the large width of the fluid receiving portion) allows the fluid receiving portion to receive urine that was not initially absorbed by the fluid permeable membrane. However, it is noted that the fluid collection devices disclosed herein may be used in connection with female urethras or non-buried penises.

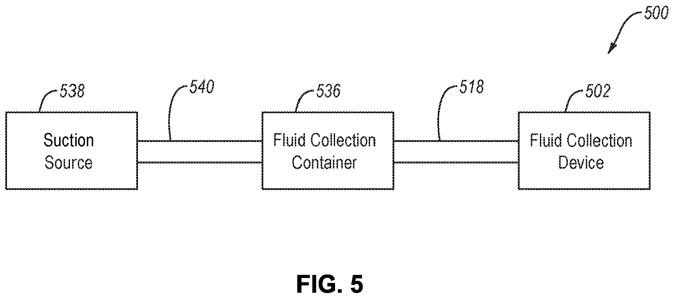

**FIG. 1A** is a top plan view of a portion of a fluid collection system 100 that includes a fluid collection device 102, according to an embodiment. The fluid collection device 102 includes a fluid impermeable barrier 104. The fluid impermeable barrier 104 defines a chamber 106 (shown in **FIG. 1B**), an opening 108, and an outlet 110. The fluid collection device 102 also includes a fluid permeable membrane 116 extending over the opening 108. The fluid permeable membrane 116 may allow fluids (*e*.*g*., urine) to enter the chamber 106 while substantially preventing a urethral opening (*e*.*g*., penis) from enter the chamber 106. The fluid collection device 102 further includes a fluid receiving portion 112 that is configured to be positioned adjacent to the urethral opening (*e.g*., a buried penis) and an longitudinal portion 114 that is configured to be positioned at and/or near a perineum of the individual. The fluid collection system 100 also includes at least one tube 118 in fluid communication with the chamber 106 via the outlet 110. For example, the tube 118 may be positioned in the outlet 110 such that a portion of the tube 118 is disposed in the chamber 106. Thus, the tube 118 may remove fluid from the chamber 106, as will be discussed in more detail with regards to **FIG. 5****.**

The fluid impermeable barrier 104 may include any fluid (*e*.*g*., urine) impermeable material. For example, the fluid impermeable barrier 104 may include a fluid impermeable polymer (*e*.*g*., silicone, polypropylene, polyethylene, polyethylene terephthalate, a polycarbonate, other elastomeric material, etc.), a metal film, natural rubber, another suitable material, or combinations thereof. As such, the fluid impermeable barrier 104 substantially prevents the fluids from passing through the fluid impermeable barrier 104. In an example, the fluid impermeable barrier 104 may be air permeable and fluid impermeable. In such an example, the fluid impermeable barrier 104 may be formed of a hydrophobic material that defines a plurality of pores. At least one or more portions of at least an outer surface of the fluid impermeable barrier 104 may be formed from a soft and/or smooth material, thereby reducing chafing. During use, a portion of the outer surface of the fluid impermeable barrier 104 may contact the wearer.

In an embodiment, forming the fluid impermeable barrier 104 from silicone and/or another elastomeric material allows the fluid impermeable barrier 104 to be bendable. Allowing the fluid impermeable barrier 104 to be bendable may make the fluid collection device 102 more comfortable to use than if the fluid impermeable barrier 104 was formed from another material. Further, as previously discussed, the fluid collection device 102 is configured to be used with a buried penis. Individuals with buried penises are often overweight and a bendable fluid impermeable barrier 104 may allow the fluid impermeable barrier 104 to bend around and/or conform to bulges caused by body fat. Bending the fluid impermeable barrier 104 around and/or conforming to the bulges caused by the body fat may inhibit fluids from avoiding the opening 108 and flowing around the fluid impermeable barrier 104.

The fluid impermeable barrier 104, in part, defines and forms the fluid receiving portion 112 and the longitudinal portion 114. For example, the fluid impermeable barrier 104 may be formed to exhibit the size and shape of the fluid receiving portion 112 and the longitudinal portion 114. However, it is noted that the other components of the fluid collection device 102 also define and form the fluid receiving portion 112 and the longitudinal portion 114. For example, the components of the fluid collection device 102 that are disposed in the chamber 106 (*e*.*g*., the fluid permeable membrane 116 and the fluid permeable support 130) may also support the fluid impermeable barrier 104 such that the fluid impermeable barrier 104 forms the shape of the fluid receiving portion 112 and the longitudinal portion 114.

As previously discussed, the fluid collection device 102 exhibits a length L_{D} extending from a distal tip 126 of the longitudinal portion 114 to an opposing portion of the fluid receiving portion 112. In the illustrated embodiment, the opposing portion of the fluid receiving portion 112 is the outlet 110. The length L_{D} of the fluid collection device 102 may be selected based on at least the size of the individual (*e.g.,* the size of the individual's groin), the type of urethral opening that the fluid collection device 102 is positioned adjacent to (*e.g.,* a buried penis may require a larger fluid receiving portion 112 than a female urethral opening due to at least the various angles that the buried penis may emit urine), and the method used to attach the longitudinal portion 114 to the individual (*e.g.,* using an adhesive may allow for a smaller longitudinal portion 114). In other words, the length L_{D} of the fluid collection device 102 may vary from one embodiment to the next. However, generally, the length L_{D} of the fluid collection device 102 may be greater than about 10 cm, greater than about 12 cm, greater than about 14 cm, greater than about 16 cm, greater than about 18 cm, greater than about 20 cm, greater than about 22 cm, greater than about 24 cm, greater than about 26 cm, greater than about 28 cm, greater than about 30 cm, greater than about 33.5 cm, greater than about 35 cm, greater than about 37.5 cm, greater than about 40 cm, greater than about 45 cm, or in ranges of about 10 cm to about 14 cm, about 12 cm to about 16 cm, about 14 cm to about 18 cm, about 16 cm to about 20 cm, about 18 cm to about 22 cm, about 20 cm to about 24 cm, about 22 cm to about 26 cm, about 24 cm to about 28 cm, about 26 cm to about 30 cm, about 28 cm to about 32.5 cm, about 30 cm to about 35 cm, about 32.5 cm to about 37.5 cm, about 35 cm to about 40 cm, or about 37.5 cm to about 45 cm. The length L_{D} of the fluid collection device 102 may be selected based on the size of the individual, the size of the penis of the individual, the length of the perineum of the individual, and the shape of the opening 108. For example, individuals that are larger (*e.g.,* have larger penises, longer perineums, etc.) may require or be able to accommodate a fluid collection device 102 exhibit a larger length L_{D} (*e.g*., greater than about 15 cm, greater than about 20 cm, or greater than about cm) than smaller patients. Further increasing the length L_{D} of the fluid collection device 102 may increase the amount of fluids received by the fluid collection device 100 and the amount of fluids that may be held within the chamber 106. However, some individuals (*e.g.,* smaller individuals) may be unable to use a fluid collection device 102 exhibiting longer lengths L_{D}.

As previously discussed, the fluid receiving portion 112 is configured to be positioned adjacent to a urethral opening of an individual, such as a buried penis, and to receive fluids from the urethral opening. As such, the fluid receiving portion 112 at least partially defines the opening 108. For example, the fluid receiving portion 112 defines all of or a substantial majority of the opening 108. Since the fluid receiving portion 112 defines the opening 108, positioning the fluid receiving portion 112 adjacent to the urethral opening of the individual also positions the opening 108 adjacent to the urethral opening. Positioning the opening 108 adjacent to the urethral opening increases the percentage of fluid that is discharged from the urethral opening that both reaches the opening 108 (*e.g.,* contact the fluid permeable membrane 116) and enters the chamber 106.

The fluid receiving portion 112 exhibits a shape that allows the fluid receiving portion 112 to be positioned adjacent to the urethral opening. Generally, the front face 120 of the fluid receiving portion 112 exhibits a shape that allows the fluid receiving portion 112 to abut the urethral opening. For example, the front face 120 may exhibit a generally planar topography or may have a concave curved topography which allows the front face 120 to conform to the groin of the individual since the groin is generally planar or has a convex curvature.

The fluid receiving portion 112 may also exhibit a shape that is configured to increase the size of the opening 108. For example, increasing the size of the fluid receiving portion 112 may allow the fluid receiving portion 112 to define a larger opening 108. The larger opening 108 allows the opening 108 to receive fluids that is emitted from a buried penis at different directions. Further, the larger opening 108 allows the opening 108 to receive fluid that would have otherwise leaked between the fluid impermeable barrier 104 and the skin of the individual. The fluid receiving portion 112 exhibits a generally triangular shape. The generally triangular shape may allow the fluid receiving portion 112 to correspond to the shape of the groin that surrounds the urethral opening since the groin exhibits a generally triangular shape, especially when the thighs of the individual contact each other (which is common for obese individuals). As such, the generally triangular shape of the fluid receiving portion 112 may allow the fluid receiving portion 112 to exhibit the maximum possible size without minimally uncomfortably pressing into the individual.

When the fluid receiving portion 112 exhibits the generally triangular shape, the fluid receiving portion 112 will include three edges 122 and at least two vertices 124 (the longitudinal portion 114 will extend from and obscure a vertex). In the illustrated embodiment, the edges 122 may be convexly curved which may at least one of cause the edges 122 to slightly press into the individual to position and secure the fluid receiving portion 112 to the groin or to conform to the groin of the individual when the edges of the groin are convex. The convex curvature of the edges 122 may also increase the volume of the chamber 106 which, in turn, increases the volume of fluid that may be held in the chamber 106 at any given time. However, it is noted that the edges 122 may be straight or convexly curved (*e.g.,* convexly curved edges 122 may be used with extremely obese individuals). Generally, any exposed vertices 124 (*e.g.,* any vertex that the longitudinal portion 114 does not extend from) may be rounded which may inhibit the vertices 124 from causing discomfort.

In an embodiment, as illustrated, the opening 108 exhibits a shape that corresponds to the shape of the fluid receiving portion 112 which allows the opening 108 to exhibit the largest possible size. However, it is noted that the opening 108 may exhibit a shape that is different than the shape of the fluid receiving portion 112. For example, while the fluid receiving portion 112 exhibits a generally triangular shape, the opening 108 may exhibit a generally circular shape. The generally circular shape of the opening 108 may allow the fluid receiving portion 112 to be positioned such that the distance from the urethral opening to the edge of the opening 108 is substantially constant since smaller distances from the urethral opening to the edge of the opening 108 are more likely to induce fluid leaks.

The fluid receiving portion 112 exhibits a width that is measured perpendicularly to the length L_{D} of the fluid collection device 102. The fluid receiving portion 112 exhibits a maximum width W_{FMax}. The maximum width W_{FMax} of the fluid receiving portion 112 may be selected based on the size of the individual (*e.g.,* the size of the individual's groin) and the type of urethral opening that the fluid collection device 102 is positioned adjacent to (*e.g.,* a buried penis may require a larger fluid receiving portion 112 than a female urethral opening due to at least the various angles that the buried penis may emit urine). In other words, the maximum width W_{FMax} of the fluid receiving portion 112 may be selected based on the application of the fluid collection device 102. The maximum width W_{FMax} may be at least about 7.5 cm, at least about 10 cm, at least about 12 cm, at least about 14 cm, at least about 16 cm, at least about 18 cm, at least about 20 cm, at least about 22 cm, at least about 24 cm, at least about 26 cm, at least about 28 cm, at least about 30 cm, or in ranges of about 7.5 cm to about 12 cm, about 10 cm to about 14 cm, at least 12 cm to about 16 cm, about 14 cm to about 18 cm, about 16 cm to about 20 cm, about 18 cm to about 22 cm, about 20 cm to about 24 cm, about 22 cm to about 26 cm, about 24 cm to about 28 cm, or about 26 cm to about 30 cm. The maximum width W_{FMax} may be selected based on the size of the individual and, more particularly, the space between the thighs of the patient. For example, the maximum width W_{FMax} may be selected to be at or near the maximum width W_{FMaz} that an individual may accommodate since, generally, increasing the maximum width W_{FMax} may increase the amount of fluids that are received by the fluid collection device 100. In the illustrated embodiment, the maximum width W_{FMax} is spaced from the vertices 124 due to the convex shape of the edges 122. However, it is noted that the maximum width W_{FMax} may be located at the vertices 124.

The fluid impermeable barrier 104 exhibits a thickness that is micrometer sized (*e.g.,* about 100 µm to about 1000 µm) to a few millimeters (*e.g.,* about 1 mm to about 5 mm). As such, the portion of the chamber 106 that form part of the fluid receiving portion 112 may exhibit maximum width (not shown, obscured) that is comparable to the maximum width W_{FMax}. For example, the portion of the chamber 106 that form part of the fluid receiving portion 112 may exhibit a maximum width that is at least about 7 cm, at least about 10 cm, at least about 12 cm, at least about 14 cm, at least about 16 cm, at least about 18 cm, at least about 20 cm, at least about 22 cm, at least about 24 cm, at least about 26 cm, at least about 28 cm, at least about 30 cm, or in ranges of about 7.5 cm to about 12 cm, about 10 cm to about 14 cm, at least 12 cm to about 16 cm, about 14 cm to about 18 cm, about 16 cm to about 20 cm, about 18 cm to about 22 cm, about 20 cm to about 24 cm, about 22 cm to about 26 cm, about 24 cm to about 28 cm, or about 26 cm to about 30 cm. As previously discussed, the maximum width of the chamber 106 may be selected based on the maximum width W_{FMax}. The maximum width of the chamber 106 that forms part of the fluid receiving portion 112 may be selected using the same factors as the maximum width W_{FMax}. The maximum width of the chamber 106 may also be selected based on the thickness of the fluid impermeable barrier 104 and the desired amount of fluid that may be held in the chamber 106.

As previously discussed, the fluid collection device 102 includes an longitudinal portion 114 that extends from the fluid receiving portion 112. As illustrated, the longitudinal portion 114 extends from a vertex of the generally triangular shape of the fluid receiving portion 112. The longitudinal portion 114 is distinguishable from the fluid receiving portion 112 based on the widths thereof. For example, in the illustrated embodiment, the width of the fluid receiving portion 112 generally decreases from the maximum width W_{FMax} to the longitudinal portion 114 while the thickness of the longitudinal portion 114 remains substantially constant from the intersection of the fluid receiving portion 112 and the longitudinal portion 114 along at least a portion of a length of the longitudinal portion 114.

The longitudinal portion 114 is configured to be positioned at and/or near the perineum of an individual. In other words, the longitudinal portion 114 is configured to be positioned between the thighs of the individual. Due to the limited space at and/or near the perineum of the individual, the width of the longitudinal portion 114 is generally less than the width of the fluid receiving portion 112. However, it is noted that the width of the longitudinal portion 114 may be greater than localized regions of the fluid receiving portion 112 (*e.g.,* the width of the longitudinal portion 114 is greater than the width of the outlet 110 and the portions of the fluid receiving portion 112 about the outlet 110). The longitudinal portion 114 exhibits a maximum width W_{LMax} that is about 1 cm to about 7.5 cm, such as in ranges of about 1 cm to about 2 cm, about 1.5 cm to about 2.5 cm, about 2 cm to about 3 cm, about 2.5 cm to about 3.5 cm, about 3 cm to about 4 cm, about 3.5 cm to about 4.5 cm, about 4 cm to about 5 cm, about 4.5 cm to about 5.5 cm, about 5 cm to about 6 cm, about 5.5 cm to about 6.5 cm, about 6 cm to about 7 cm, or about 6.5 cm to about 7.5 cm. The maximum width W_{LMax} may be selected based on the required attachment force required to maintain the fluid receiving portion 112 adjacent to the urethral opening of the individual since, generally, increasing the width of the longitudinal portion 114 increases the attachment force. For example, strongly attaching the longitudinal portion 114 to the individual, such as with an adhesive, may allow the longitudinal portion 114 to exhibit a smaller maximum width W_{LMax} than if the longitudinal portion 114 relies on friction from the perineum and/or thighs (e.g., interference fit) to hold the longitudinal portion 114 in place. Also, the maximum width W_{LMax} may be selected based on the size of fluid receiving portion 112 since increasing the size of the fluid receiving portion 112 requires a larger attachment force to maintain the position of the fluid receiving portion 112. The maximum width W_{LMax} may be selected based on the size of the perineum and/or the space between the thighs of the individual using the fluid collection device 100 since the longitudinal portion 114 may be at least partially positioned adjacent to the perineum and/or between the thighs.

As previously discussed, the fluid impermeable barrier 104 exhibits a thickness that is micrometer sized to a few millimeters. As such, the portion of the chamber 106 that form part of the longitudinal portion 114 exhibits maximum width (not shown, obscured) that is comparable to the maximum width W_{LMax}. For example, the portion of the chamber 106 that form part of the longitudinal portion 114 may exhibit a maximum width that is about 0.5 cm to about 7.5 cm, such as in ranges of about 1 cm to about 2 cm, about 1.5 cm to about 2.5 cm, about 2 cm to about 3 cm, about 2.5 cm to about 3.5 cm, about 3 cm to about 4 cm, about 3.5 cm to about 4.5 cm, about 4 cm to about 5 cm, about 4.5 cm to about 5.5 cm, about 5 cm to about 6 cm, about 5.5 cm to about 6.5 cm, about 6 cm to about 7 cm, or about 6.5 cm to about 7.5 cm. The maximum width of the chamber 106 that forms part of the longitudinal portion 114 may be selected using the same factors as the maximum width W_{LMax}. The maximum width of the chamber 106 may also be selected based on the thickness of the fluid impermeable barrier 104 and the desired amount of fluid that may be held in the chamber 106.

In the illustrated embodiment, the longitudinal portion 114 includes two edges 128 extending from the fluid receiving portion 112 and the width of the longitudinal portion 114 may be measured between the two edges 128. The two edges 128 are illustrated as being substantially straight such that the width of the longitudinal portion 114 remains constant or substantially constant. However, it is noted that the two edges 128 may be curved or exhibit another type of contour. In an example, the two edges 128 may be convexly curved to increase contact between the longitudinal portion 114 and the thighs of the individual to improve the attachment (*e.g.,* interference fit) between the longitudinal portion 114 and the thighs. In an example, the two edges 128 may be concavely curved to better accommodate bulges of body fat thereby making the longitudinal portion 114 more comfortable. In an example, at least a portion of the two edges 128 may be tapered to better fit between the thighs of the individual.

The longitudinal portion 114 also exhibits a length L_{L} extending between the distal tip 126 of the longitudinal portion 114 to the intersection between the fluid receiving portion 112 and the longitudinal portion 114. The length L_{L} is measured perpendicularly to the length L_{D} of the fluid collection device 102. The length L_{L} may be at least about 5 cm, at least about 7.5 cm, at least about 10 cm, at least about 12 cm, at least about 14 cm, at least about 16 cm, at least about 18 cm, at least about 20 cm, at least about 22 cm, at least about 24 cm, at least about 26 cm, at least about 28 cm at least about 30 cm, or in ranges of about 5 cm to about 10 cm, about 7.5 cm to about 12 cm, 10 cm to about 14 cm, about 12 cm to about 16 cm, at least 14 cm to about 18 cm, about 16 cm to about 20, about 18 cm to about 22 cm, about 20 cm to about 24 cm, about 22 cm to about 26 cm, about 24 cm to about 28 cm, or about 26 cm to about 30 cm. Similar to the maximum width W_{LMax}, the length L_{L} of the longitudinal portion 114 may be selected based on the required attachment force required to maintain the fluid receiving portion 112 adjacent to the urethral opening of the individual since, generally, increasing the width of the longitudinal portion 114 increases the attachment force. The length L_{L} of the longitudinal portion 114 may be selected based on the size of the perineum since the longitudinal portion 114 may be at least partially positioned adjacent to the perineum.

**FIG. 1B** is a cross-sectional view of the fluid collection system 100, according to an embodiment. As shown in **FIG. 1B**, the fluid collection device 102 exhibits a substantially constant thickness t along substantially the entirety of the length L_{D} of the fluid collection device 102. However, in other embodiments, the thickness t may vary along the length L_{D}. For example, as illustrated, the thickness t may be substantially constant except near the outlet 110, near the distal tip 126, and at the opening 108. The uniform thickness of the fluid collection device 102 may allow the fluid collection device 102 to be worn discretely. However, the thickness of the fluid collection device 102 may vary. In an example, thickness of the fluid receiving portion 112 may be selected to be larger than the thickness of the longitudinal portion 114 to increase the volume of fluid that may be stored in the chamber 106. In an example, the thickness of the fluid receiving portion 112 may be selected to be smaller than the thickness of the longitudinal portion 114 to further increase the ability of the fluid collection device 102 to be worn discretely.

As previously discussed, the fluid collection device 102 may include a fluid permeable membrane 116 disposed in the chamber 106. The fluid permeable membrane 116 may extend across at least a portion (*e.g.,* all) of the opening 108. The fluid permeable membrane 116 may be configured to wick any fluid away from the opening 108 thereby allowing fluid to enter the chamber 106 and preventing the fluid from escaping the chamber 106. The permeable properties may have wicking, capillary action, diffusion, or other similar properties or processes, and are referred to herein as "permeable" and/or "wicking." Such "wicking" may not include absorption into the fluid permeable membrane. The fluid permeable membrane 116 may also wick the fluid generally towards an interior of the chamber 106. The fluid permeable membrane 116 may include any material that may wick the fluid. For example, the fluid permeable membrane 116 may include fabric, such as a gauze (*e.g.,* a silk, linen, or cotton gauze), another soft fabric, or another smooth fabric. Forming the fluid permeable membrane 116 from gauze, soft fabric, and/or smooth fabric may reduce chaffing caused by the fluid collection device 102. In an example, the fluid permeable membrane 116 may include a one way flow fabric to prevent fluid from leaking from the chamber 106 through the fluid permeable membrane 116.

The fluid collection device 102 may include a fluid permeable support 130 disposed in the chamber 106. The fluid permeable support 130 is configured to support the fluid permeable membrane 116 since the fluid permeable membrane 116 may be formed from a foldable, flimsy, or otherwise easily deformable material. For example, the fluid permeable support 130 may be positioned such that the fluid permeable membrane 116 is disposed between the fluid permeable support 130 and the fluid impermeable barrier 104. As such, the fluid permeable support 130 may support and maintain the position of the fluid permeable membrane 116. The fluid permeable support 130 may be formed from any fluid permeable material that is less deformable than the fluid permeable membrane 116. For example, the fluid permeable support 130 may include a porous polymer (*e.g.,* nylon, polyester, polyurethane, polyethylene, polypropylene, etc.) structure (*e.g.,* spun fibers such as spun nylon fibers) or an open cell foam. The fluid permeable support 130 may also wick any fluids that enter the chamber 106 such that, for example, the fluids are removed from the opening 108 and directed towards the tube 118. In an example, the fluid permeable support 130 may be omitted from the fluid collection device 102.

In an embodiment, at least one of the fluid permeable membrane 116 or the fluid permeable support 130 may be a wicking material configured to wick any of the bodily fluids away from the opening 108, thereby preventing bodily fluids from escaping the chamber 106. The wicking material may not include absorption of the bodily fluids into the wicking material. Put another way, substantially no absorption of the bodily fluids into the wicking material may take place after the wicking material is exposed to the bodily fluids. While no absorption is desired, the term "substantially no absorption" may allow for nominal amounts of absorption of the bodily fluids into the wicking material (*e.g.,* absorbency), such as about 10 wt% of the dry weight of the wicking material, about 7 wt%, about 5 wt%, about 3 wt%, about 2 wt%, about 1 wt%, or about 0.5 wt% of the dry weight of the wicking material.

In an embodiment, the fluid permeable membrane 116 and the fluid permeable support 130 may at least substantially completely fill the portions of the chamber 106 that are not occupied by the tube 118. In an embodiment, the fluid permeable membrane 116 and the fluid permeable support 130 do not substantially completely fill the portions of the chamber 106 that are not occupied by the tube 118. In such an embodiment, the fluid collection device 102 includes a reservoir 132 disposed in the chamber 106. The reservoir 132 is a substantially unoccupied portion of the chamber 106. The fluids that are in the chamber 106 may flow through the fluid permeable membrane 116 and/or fluid permeable support 130 to the reservoir 132. The reservoir 132 may store at least some of the fluids therein.

In an embodiment, the inlet of the tube 118 (*e.g.,* distal tip of the tube 118) may be disposed in or near the reservoir 132. For example, the inlet of the tube 118 may be positioned in the reservoir 132 (as illustrated) or may be flush with the portions of the fluid permeable membrane 116 and/or fluid permeable support 130 that partially define the reservoir 132. In an embodiment, the inlet of the tube 118 may be spaced from the reservoir 132, for example, by at least a portion of the fluid permeable support 130.

In an example, the reservoir 132 may be located at and/or near the distal tip 126. However, the reservoir 132 may be located at different locations in the chamber 106. For example, the reservoir 132 may be located at and/or near the outlet 110. In another example, fluid collection device 102 may include multiple reservoirs, such as a first reservoir that is located at and/or near the distal tip 126 and a second reservoir at and/or near the outlet 110. In another example, the fluid permeable support 130 is spaced from at least a portion of the tube 118 and the reservoir 132 may be the space between the fluid permeable support 130 and the tube 118.

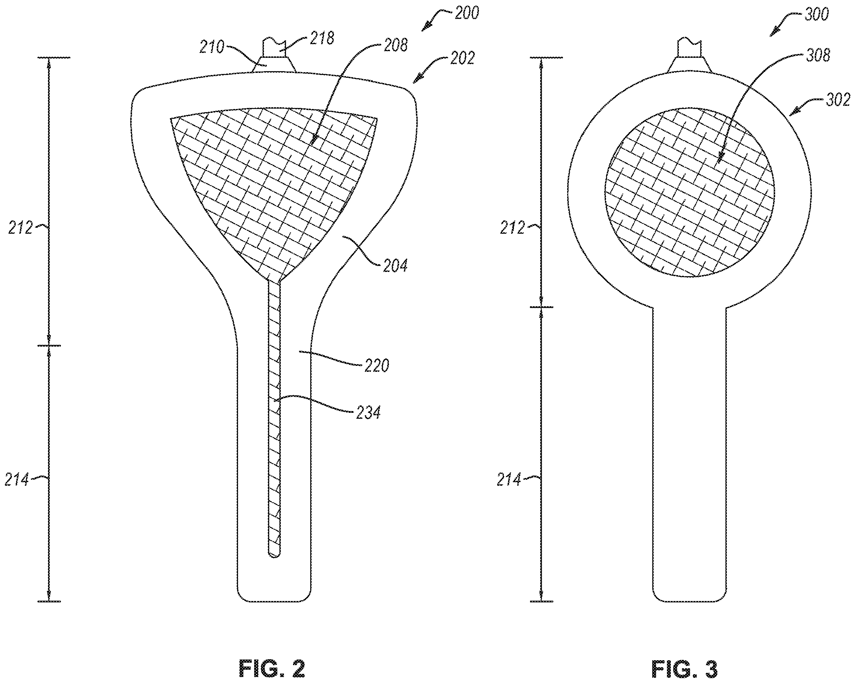

**FIG. 2** is a top plan view of a portion of a fluid collection system 200, according to an embodiment. Except as otherwise disclosed herein, the fluid collection system 200 is the same or substantially similar to any of the fluid collection systems disclosed herein. For example, the fluid collection system 200 may include a fluid collection device 202. The fluid collection device 202 includes a fluid impermeable barrier 204 defining a chamber (not shown), an opening 208, and an outlet 210 configured to receive a tube 218. The fluid collection device 202 also includes a fluid receiving portion 212 that defines the opening 208 and an longitudinal portion 214 extending from the fluid receiving portion 212.

The fluid collection device 202 also includes a slit 234. The slit 234 extends from the opening 208 and into the longitudinal portion 214. As such, the slit 234 is at least partially defined by the longitudinal portion 214 (*e.g.,* the portion of the fluid impermeable barrier 204 that forms part of the fluid receiving portion 212). The slit 234 may be configured to receive fluid (*e.g.,* urine or other bodily fluid) that is not received by the opening 208. For example, fluid that is not received by the opening 208 may trickle down the perineum of the individual since the groin channels the fluid towards the perineum when the individual is lying on the individual's back, sitting up, or standing. The slit 234 is positioned at or near the perineum of the individual since the longitudinal portion 214 is positioned at or near the perineum. This position of the slit 234 allows the slit 234 to receiving at least some of the fluid that trickles down the perineum.

In an embodiment, the slit 234 exhibits a width that is relatively narrow relative to the width of the longitudinal portion 214. For example, the slit 234 exhibits a width that is at most about 50% (*e.g.,* at most about 40%, at most about 30%, at most about 20%, or in ranges of about 5% to about 20%, about 15% to about 30 %, about 20% to about 40%, or about 30% to about 50%) the corresponding (*i.e.,* parallel and overlapping) width of the longitudinal portion 214. The relatively narrow width of the slit 234 provides sufficient surface area for an adhesive to be applied to the front face 220 of the longitudinal portion 214. The width of the slit 234 may be selected based on the corresponding width of the longitudinal portion 214 because, generally, increasing the corresponding width of the longitudinal portion 214 allows for the width of the slit 234 to occupy a greater percentage of the corresponding width. However, it is noted that in some embodiments, the slit 234 may exhibit a width that is more than about 50% (*e.g.,* 50% to about 75% or about 50% to about 60%) the corresponding width of the longitudinal portion 214.

In an embodiment, the slit 234 extends along a majority of the length of the longitudinal portion 214. In such an embodiment, the slit 234 may receive more fluid than if the slit 234 extending only along a small percentage (*e.g.,* less than 50%) of the length of the longitudinal portion 214. However, in some embodiments, the slit 234 may only extend along a small percentage of the length of the longitudinal portion 214.

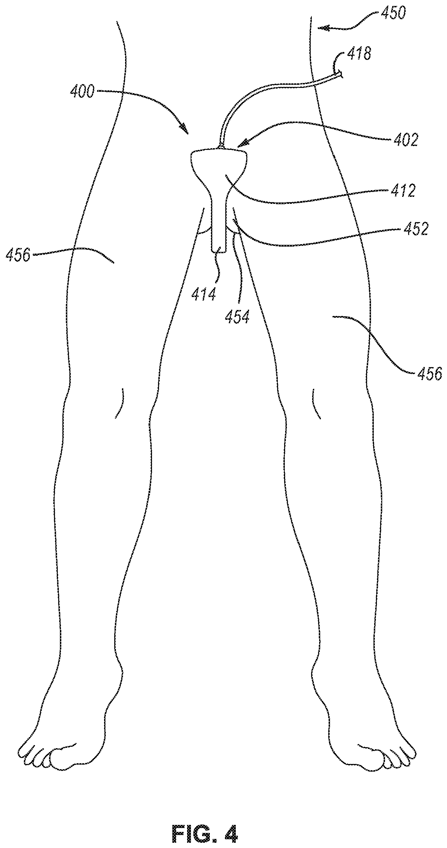

As previously discussed, the fluid collection devices disclosed herein may be positioned on an individual. **FIG. 4** is a front view of a urine collection system 400 worn by an individual 450, according to an embodiment. Except as otherwise disclosed herein, the urine collection system 400 is the same or substantially similar to any of the urine collection systems disclosed herein. For example, the urine collection system 400 may include a fluid collection device 402 including a fluid receiving portion 412 and an longitudinal portion 414. In the illustrated embodiment, the fluid collection device 402 is the same or substantially similar to the fluid collection device 102 or 202 of **FIGS. 1A-2****.** However, it is noted that the fluid collection device 402 may be the same or similar to any of the other fluid collection devices disclosed herein.

The individual 450 includes a groin 452, a perineum 454, and two thighs 456. In the illustrated embodiment, the thighs 456 of the individual 450 are illustrated as being separated such that the view of the fluid collection device 402 is unobstructed. However, there is no requirement that the thighs 456 of the individual 450 are separated and, in some embodiments, the thighs 456 of the individual 450 may need to contact each other or at least be positioned proximate to each other to maintain the position of the fluid collection device 402.

The fluid collection device 402 is positioned on the individual 450 such that the fluid receiving portion 412 is positioned adjacent to the urethral opening (not shown, obscured) of the individual 450. In other words, the fluid receiving portion 412 is positioned adjacent to the groin 452 of the individual 450. The longitudinal portion 414 is then positioned against and/or near the perineum 454 of the individual 450. This allows the fluid collection device 402 to receive fluid (*e.g.,* urine) from the individual 450 and the maintain the position of the fluid collection device 402 relative to the individual 450, as discussed in more detail above.

**FIG. 5** is a schematic illustrating of a fluid collection system 500, according to an embodiment. The fluid collection system 500 includes a fluid collection device 502 that is the same or substantially similar to any of the fluid collection devices disclosed herein. For example, the fluid collection device 502 may include a fluid receiving portion and an longitudinal portion. The fluid collection system 500 also include a fluid collection container 536 that is in indirectly fluid communication with the fluid collection device 502 via at least one first tube 518 (*e.g.,* tube 118 or 218 of **FIGS. 1A-2**). The fluid collection container 536 may be positioned downstream from the fluid collection device 502. The fluid collection system 500 also includes a suction source 538 that is configured to apply a suction force (*e.g.,* a continuous suction force) to the chamber of the fluid collection device 502 thereby removing fluid from the chamber and depositing the fluid in the fluid collection container 536. In the illustrated embodiment, the suction source 538 is vacuum source. In such an embodiment, the suction source 538 is positioned downstream from the fluid collection container 536 and may be in fluid communication with the fluid collection container 536 via at least one second tube 540. However, in an embodiment, the suction source 538 is a gas source that is positioned upstream from the fluid collection device 502. Examples of fluid collection systems that include gas sources are disclosed in PCT Application No. PCT/US2019/029609 filed on April 29, 2019.

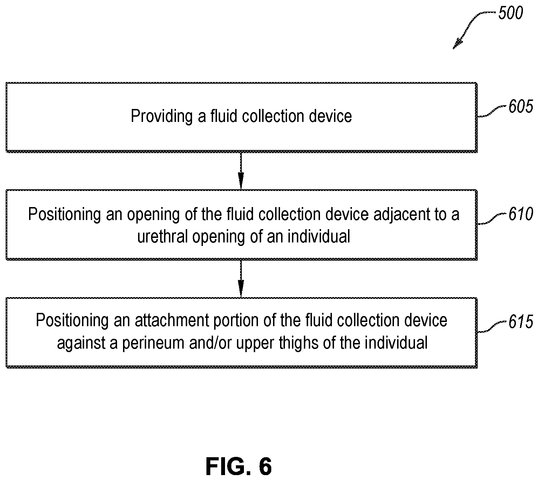

**FIG. 6** is a flow diagram of a method 600 to use any of the fluid collection devices and/or fluid collection systems disclosed herein, according to an embodiment. The method 600 may include act 605, which recites "providing a fluid collection device." Act 605 may be followed by act 610, which recites "positioning an opening of the fluid collection device adjacent to a urethral opening of an individual." Act 610 may be followed by act 615, which recites "positioning an longitudinal portion of the fluid collection device against a perineum and/or upper thighs of the individual."

Acts 605, 610, 615 of the method 600 are for illustrative purposes. For example, the act 605, 610, 615 of the method 600 may be performed in different orders, split into multiple acts, modified, supplemented, or combined. In an example, one or more of the acts 605, 610, 615 of the method 600 may be omitted from the method 600.

Act 605 recites "providing a fluid collection device." Act 605 includes providing any of the fluid collection devices disclosed herein. For example, act 605 may include providing a fluid collection device that includes a fluid impermeable barrier defining a chamber, an opening, and an outlet. The fluid collection device may also include a fluid permeable membrane extending across the opening. The fluid collection device may also include a fluid receiving portion that includes a portion of the fluid impermeable barrier that at least partially defines the opening and an longitudinal portion extending from the fluid receiving portion.

Act 610 recites "positioning an opening of the fluid collection device adjacent to a urethral opening of an individual." In an example, act 610 may include positioning the opening adjacent to a buried penis. In an example, act 610 may include positioning the opening adjacent to a female urethral opening or a non-buried penis.

Act 615 recites "positioning an longitudinal portion of the fluid collection device against a perineum and/or upper thighs of the individual." In an example, act 615 includes attaching the longitudinal portion against the perineum and/or upper thighs with an adhesive. In an example, act 615 includes attaching the longitudinal portion against the perineum and/or upper thighs by merely positioning the longitudinal portion at or near the perineum and allowing the contact between longitudinal portion and the thighs to hold the longitudinal portion in its position.

The method 600 may also include receiving fluids (e.g., urine) into the chamber. For example, a urethral opening may emit urine that contacts the fluid permeable membrane and the fluid permeable membrane may wick the urine into the chamber. A suction force may be applied to the chamber which may facilitate the fluid permeable membrane wicking the urine into the chamber. The urine that is wicked into the chamber may flow from the fluid permeable membrane towards at least one inlet of a tube that is disposed in the chamber. For example, the urine may flow from the fluid permeable membrane, through the fluid permeable support, into the fluid reservoir, and into the tube.

As previously discussed, a suction force may be applied to the chamber. The suction force may be a continuous suction force or a discontinuous suction force. The suction force may pull the fluids that are present in the chamber into the tube, through the tube, to the fluid collection container, and deposit the fluid in the fluid collection container.

Terms of degree (*e.g.,* "about," "substantially," "generally," etc.) indicate structurally or functionally insignificant variations. In an example, when the term of degree is included with a term indicating quantity, the term of degree is interpreted to mean ± 10%, ±5%, ± 2%, or 0% of the term indicating quantity. In an example, when the term of degree is used to modify a shape, the term of degree indicates that the shape being modified by the term of degree has the appearance of the disclosed shape. For instance, the term of degree may be used to indicate that the shape may have rounded corners instead of sharp corners, curved edges instead of straight edges, one or more protrusions extending therefrom, is oblong, is the same as the disclosed shape, etc.

## Claims

1. A fluid collection device (102), comprising:
a fluid impermeable barrier (104) defining a chamber (106), an opening (108), and an outlet (110);
a fluid permeable membrane (116) extending across the opening;
a fluid receiving portion (112) including a portion of the fluid impermeable barrier that at least partially defines the opening; and
a longitudinal portion (114) extending from the fluid receiving portion;
wherein the fluid collection device exhibits a maximum length measured from a distal tip of the longitudinal portion to an opposing portion of the fluid receiving portion;
wherein the fluid receiving portion exhibits a first maximum width measured perpendicular to the maximum length, and the longitudinal portion exhibits a second maximum width measured parallel to the first maximum width, and wherein the first maximum width is greater than the second maximum width;
wherein the opening is for positioning adjacent to the urethral opening of an individual;
wherein the longitudinal portion has an oblong form and is for positioning against the perineum of the individual;
wherein the fluid receiving portion exhibits a generally triangular shape;
wherein the longitudinal portion extends from a vertex of the generally triangular shape; and
**characterised in that**
the outlet (110) is in the opposing portion of the fluid receiving portion (112).

2. The fluid collection device of claim 1, wherein the opening exhibits a generally circular shape.

3. The fluid collection device as claimed in claim 1 or 2, wherein the longitudinal portion exhibits a length measured from the distal tip of thereof to a intersection of the longitudinal portion and the fluid receiving portion that is at least 10 cm and/or wherein the second maximum width is from 2 cm to 6 cm.

4. The fluid collection device as claimed in any one of the preceding claims, wherein the longitudinal portion at least partially defines a slit (234) extending from the opening.

5. The fluid collection device of any one of the preceding claims, further comprising a tube (118) positioned through the outlet.

6. The fluid collection device of claim 5, wherein an inlet of the tube is disposed in a substantially unoccupied fluid reservoir (132).

7. The fluid collection device of claim 6, wherein the longitudinal portion defines the substantially unoccupied reservoir (132) at or near the distal tip thereof.

8. A fluid collection system, comprising:
the fluid collection device on any one of claims 1-6;
a fluid collection container (536) in fluid communication with and positioned downstream from the fluid collection device;
a suction source (538) in fluid communication with the fluid collection device and the fluid collection container; and
at least one tube (518) that allows the fluid collection device to be in fluid communication with the fluid collection container and the suction source.

9. The fluid collection system of claim 8, wherein the suction source is configured to provide a continuous suction force to the fluid collection device.

10. The fluid collection system of claim 8, wherein the suction source is a vacuum source positioned downstream from the fluid collection container.

## Patentansprüche

1. Fluidsammelvorrichtung (102), umfassend:
eine fluidundurchlässige Barriere (104), die eine Kammer (106), eine Öffnung (108) und einen Auslass (110) definiert;
eine fluiddurchlässige Membran (116), die sich über die Öffnung erstreckt;
einen Fluidaufnahmeabschnitt (112), der einen Abschnitt der fluidundurchlässigen Barriere einschließt, der zumindest teilweise die Öffnung definiert; und
einen Längsabschnitt (114), der sich von dem Fluidaufnahmeabschnitt erstreckt;
wobei die Fluidsammelvorrichtung eine maximale Länge aufweist, gemessen von einer distalen Spitze des Längsabschnitts bis zu einem gegenüberliegenden Abschnitt des Fluidaufnahmeabschnitts;
wobei der Fluidaufnahmeabschnitt eine erste maximale Breite aufweist, gemessen senkrecht zur maximalen Länge, und der Längsabschnitt eine zweite maximale Breite aufweist, gemessen parallel zur ersten maximalen Breite, und wobei die erste maximale Breite größer ist als die zweite maximale Breite;
wobei die Öffnung zum Positionieren angrenzend an die Harnröhrenöffnung eines Individuums vorgesehen ist;
wobei der Längsabschnitt eine längliche Form aufweist und zum Positionieren gegen das Perineum des Individuums vorgesehen ist;
wobei der Fluidaufnahmeabschnitt eine allgemein dreieckige Form aufweist;
wobei sich der Längsabschnitt von einem Scheitelpunkt der allgemein dreieckigen Form erstreckt; und
**dadurch gekennzeichnet, dass**
der Auslass (110) sich in dem gegenüberliegenden Abschnitt des Fluidaufnahmeabschnitts (112) befindet.

2. Fluidsammelvorrichtung nach Anspruch 1, wobei die Öffnung eine allgemein kreisförmige Form aufweist.

3. Fluidsammelvorrichtung nach Anspruch 1 oder 2, wobei der Längsabschnitt eine Länge aufweist, gemessen von seiner distalen Spitze bis zu einem Schnittpunkt des Längsabschnitts und des Fluidaufnahmeabschnitts, die mindestens 10 cm beträgt und/oder wobei die zweite maximale Breite 2 cm bis 6 cm beträgt.

4. Fluidsammelvorrichtung nach einem der vorstehenden Ansprüche, wobei der Längsabschnitt zumindest teilweise einen Schlitz (234) definiert, der sich von der Öffnung erstreckt.

5. Fluidsammelvorrichtung nach einem der vorstehenden Ansprüche, weiter umfassend ein durch den Auslass positioniertes Rohr (118).

6. Fluidsammelvorrichtung nach Anspruch 5, wobei ein Einlass des Rohrs in einem im Wesentlichen nicht belegten Fluidreservoir (132) angeordnet ist.

7. Fluidsammelvorrichtung nach Anspruch 6, wobei der Längsabschnitt das im Wesentlichen nicht belegte Reservoir (132) an oder in der Nähe der distalen Spitze desselben definiert.

8. Fluidsammelsystem, umfassend:
die Fluidsammelvorrichtung nach einem der Ansprüche 1-6;
einen Fluidsammelbehälter (536), der in Fluidverbindung mit der Fluidsammelvorrichtung steht und stromabwärts von dieser positioniert ist;
eine Saugquelle (538), die in Fluidverbindung mit der Fluidsammelvorrichtung und dem Fluidsammelbehälter steht; und
mindestens ein Rohr (518), das es der Fluidsammelvorrichtung ermöglicht, mit dem Fluidsammelbehälter und der Saugquelle in Fluidverbindung zu stehen.

9. Fluidsammelsystem nach Anspruch 8, wobei die Saugquelle konfiguriert ist zum Ausüben einer kontinuierlichen Saugkraft auf die Fluidsammelvorrichtung.

10. Fluidsammelsystem nach Anspruch 8, wobei die Saugquelle eine stromabwärts vom Fluidsammelbehälter positionierte Vakuumquelle ist.

## Revendications

1. Dispositif de collecte de liquide (102), comprenant :
une barrière imperméable aux liquides (104) définissant une chambre (106), une ouverture (108) et un orifice de sortie (110) ;
une membrane perméable aux liquides (116) s'étendant à travers l'ouverture ;
une partie de réception de liquide (112) incluant une partie de la barrière imperméable aux liquides qui définit au moins partiellement l'ouverture ; et
une partie longitudinale (114) s'étendant à partir de la partie de réception de liquide ;
dans lequel le dispositif de collecte de liquide présente une longueur maximale mesurée à partir d'une extrémité distale de la partie longitudinale jusqu'à une partie opposée de la partie de réception de liquide ;
la partie de réception de liquide présente une première largeur maximale mesurée perpendiculairement à la longueur maximale, et la partie longitudinale présente une seconde largeur maximale mesurée parallèlement à la première largeur maximale, et dans lequel la première largeur maximale est supérieure à la seconde largeur maximale ;
dans lequel l'ouverture est destinée à être positionnée à proximité de l'orifice urétral d'un individu ;
dans lequel la partie longitudinale présente une forme oblongue et est destinée à être positionnée contre le périnée de l'individu ;
la partie de réception de liquide présente une forme essentiellement triangulaire ;
dans laquelle la partie longitudinale s'étend à partir d'un sommet de la forme essentiellement triangulaire ; et
**caractérisé en ce que**
l'orifice de sortie (110) est dans la partie opposée de la partie de réception de liquide (112).

2. Dispositif de collecte de liquide selon la revendication 1, dans lequel l'ouverture présente une forme essentiellement circulaire.

3. Dispositif de collecte de liquide selon la revendication 1 ou la revendication 2, dans lequel la partie longitudinale présente une longueur mesurée entre son extrémité distale et l'intersection de la partie longitudinale et la partie de réception de liquide qui est au moins de 10 cm, et/ou dans lequel la seconde largeur maximale est de 2 cm à 6 cm.

4. Dispositif de collecte de liquide selon l'une quelconque des revendications précédentes, dans lequel la partie longitudinale définit au moins partiellement une fente (234) s'étendant à partir de l'ouverture.

5. Dispositif de collecte de liquide selon l'une quelconque des revendications précédentes, comprenant en outre un tube (118) positionné à travers l'orifice de sortie.

6. Dispositif de collecte de liquide selon la revendication 5, dans lequel un orifice d'entrée du tube est situé dans un réservoir de liquide sensiblement inoccupé (132).

7. Dispositif de collecte de liquide selon la revendication 6, dans lequel la partie longitudinale définit le réservoir sensiblement inoccupé (132) à son extrémité distale ou à proximité de celle-ci.

8. Système de collecte de liquide, comprenant :
le dispositif de collecte de liquide selon l'une quelconque des revendications 1 à 6 ;
un réservoir de collecte de liquide (536) en communication fluidique avec le dispositif de collecte de liquide et positionné en aval de celui-ci ;
une source d'aspiration (538) en communication fluidique avec le dispositif de collecte de liquide et le réservoir de collecte de liquide ; et
au moins un tube (518) qui permet au dispositif de collecte de liquide d'être en communication fluidique avec le réservoir de collecte de liquide et la source d'aspiration.

9. Système de collecte de liquide selon la revendication 8, dans lequel la source d'aspiration est configurée de manière à fournir une force d'aspiration continue au dispositif de collecte de liquide.

10. Système de collecte de liquide selon la revendication 8, dans lequel la source d'aspiration est une source de vide positionnée en aval du récipient de collecte de liquide.
